# EUROPÄISCHE PATENTSCHRIFT

(11) **EP 1 312 317 B1**
(45) Veröffentlichungstag und Bekanntmachung des Hinweises auf die Patenterteilung: **23.06.2004**
(21) Anmeldenummer: 01126462.9
(22) Anmeldetag: 09.11.2001
(51) Int. Cl.: A61B 19/00

(54) **Schwenkbarer Arm mit passiven Aktuatoren**
Pivotable arm with passive actuators
Bras pivotant équipé d'actionneurs passifs

(43) Veröffentlichungstag der Anmeldung: 21.05.2003
(73) Patentinhaber: BrainLAB AG, 85551 Kirchheim/Heimstetten (DE)
(72) Erfinder: Ruch, Christof, 81679 München (DE)
(74) Vertreter: Schwabe - Sandmair - Marx

(56) Entgegenhaltungen:
- US-A- 5 828 197
- US-B1- 6 231 526
- DAVIES B L ET AL: "Robotic surgery at Imperial College London" PROCEEDINGS OF THE INTERNATIONAL CONFERENCE ON SYSTEMS, MAN AND CYBERNETICS. LE TOUQUET, OCT. 17 - 20, 1993, NEW YORK, IEEE, US, Bd. 3, 17. Oktober 1993 (1993-10-17), Seiten 176-181, XP010132247 ISBN: 0-7803-0911-1
- ARNE RADETZKY: "ROBO-SIM: A simulator for minimally invasive neurosurgery using a passive manipulator" [Online] Februar 2000 (2000-02) XP002186724 Gefunden im Internet: <URL: http://www.ism-austria.at/arne/ara/robosim /robosim.html> [gefunden am 2002-01-07] * das ganze Dokument *

## Beschreibung

Die vorliegende Erfindung bezieht sich auf einen schwenkbaren Arm mit passiven Aktuatoren, insbesondere zur Verwendung auf dem Gebiet der computerunterstützten Chirurgie für stereotaktische Anwendungen.

Aus der US 5,820,623 ist ein Gelenk-Arm bekannt, welcher zum Halten und Positionieren von medizinischen Instrumenten dient. Dabei ist eine Antriebsvorrichtung mit einem ServoMotor vorgesehen, um ein Gelenkelement des Armes zu bewegen.

Aus der US 5,952,796 ist eine Vorrichtung und ein Verfahren zum Führen von Bewegungen einer Bedienperson bekannt, um zum Beispiel unter Verwendung eines Steuermotors eine Person beim Bewegen einer Last zu unterstützen.

Die US 5,704,253 offenbart eine Vorrichtung zur Führung einer Bewegung entlang einer vorgegebenen Trajektorie.

Aus der US 5,828,197 sind eine Vorrichtung und ein Verfahren zum Koppeln der Bewegung eines von einem Benutzer bewegbaren Objektes mit einem Computersystem bekannt, um zum Beispiel ein virtual reality System zu Trainingszwecken zu schaffen. In einer Ausführungsform wird ein von einem Benutzer bewegbares Objekt mit einer mechanischen Schnittstelle verbunden und das Objekt kann im dreidimensionalen Raum bewegt werden, wobei die Position des Objektes durch die mechanische Schnittstelle erfasst und mittels Sensoren in Signale umgewandelt wird, welche an eine elektronische Schnittstelle weitergeleitet werden. Ein Rechner erzeugt eine Information zur Ansteuerung von Aktuoatoren der mechanischen Schnittstelle, so dass diese Aktuatoren Kräfte erzeugen, welche auf das Objekt wirken, so dass die auf das Objekt wirkenden Kräfte beim Benutzer den Eindruck einer realistischen Simulation hervorrufen.

Davies B.L. und Hibberd R.D. beschreiben in "Robotic Surgery at Imperial College London", Proceedings of the international Conference on Systems, Man and Cybernetics. Le Touquet, Oktober 17 - 20, 1993, New York, IEEE, US, Band 3, 17. Oktober 1993, Seiten 176-181, XP010132247 ISBN: 0-7803-0911-1 einen passiven Roboterarm mit zum Beispiel elektromagnetischen Bremsen, um zum Beispiel den Arm über einen längeren Zeitraum an einer festen Stelle zu halten, um zum Beispiel die Behandlung mit radioaktiven Substanzen zu ermöglichen.

Es ist eine Aufgabe der vorliegenden Erfindung einen schwenkbaren Arm, ein System mit einem schwenkbaren Arm und ein Verfahren zur Ansteuerung eines schwenkbaren Armes vorzuschlagen, welche zur Unterstützung bei der Untersuchung oder Behandlung eines Körpers verwendet werden können.

Diese Aufgabe wird durch die in den unabhängigen Ansprüchen definierten Vorrichtungen und Verfahren gelöst. Vorteilhafte Ausführungsformen ergeben sich aus den Unteransprüchen.

Der erfindungsgemäße schwenkbare Arm ist an einem Ende an einem geeigneten Halter, wie zum Beispiel einem Tisch oder einer medizinischen Vorrichtung befestigbar und weist mindestens ein Gelenk und bevorzugt mehrere Gelenke auf, so dass der befestigte schwenkbare Arm mit mindestens einem Freiheitsgrad, bevorzugt zwei, drei, vier, fünf oder mehr Freiheitsgraden bewegt werden kann. Ein Gelenk kann hierbei eine Drehbewegung und/oder eine Kippbewegung ermöglichen, so dass zum Beispiel ein Kugellager zur Ermöglichung einer Drehbewegung oder ein Schamiergelenk zur Ermöglichung einer Kippbewegung verwendet werden können. Im Sinne der Erfindung soll zum Beispiel auch ein Teleskopelement als Gelenk verstanden werden, mit welchem eine Bewegung in axialer Richtung des Teleskopelements möglich ist. Allgemein soll der schwenkbare Arm durch Anbringen mindestens eines Gelenkes so ausgestaltet sein, dass eine Bewegung mit mindestens einem Freiheitsgrad durchgeführt werden kann. An dem mindestens einen Gelenk ist ein Sensor vorgesehen, mit welchem die Position oder Bewegung des mindestens einen Gelenkes festgestellt werden kann. Erfindungsgemäß ist an dem mindestens einen Gelenk weiterhin ein steuerbarer passiver Aktuator, zum Beispiel eine steuerbare Bremse vorgesehen, um die Bewegung des schwenkbaren Arms an dem spezifischen Gelenk freizugeben, mit einer variablen Widerstandskraft zu erschweren bzw. abzubremsen oder sogar ganz unmöglich zu machen, d.h. zum Beispiel das Gelenk in einer bestimmten Position zu arretieren. Der steuerbare passive Aktuator wird erfindungsgemäß von einer Steuervorrichtung angesteuert, welche basierend auf den Sensordaten des mindestens einen Gelenksensors die momentane Position des gesamten schwenkbaren Armes, insbesondere die Position eines mit dem schwenkbaren Arm verbundenen Instruments oder der Instrumentenspitze bestimmen kann. Erfindungsgemäß werden Daten eines mit dem Instrument zu untersuchenden oder zu behandelnden Zielobjekts, zum Beispiel eines Körpers oder Körperteils vor oder auch während der Verwendung des Armes zum Beispiel durch CT oder MRI erfasst und von der Steuervorrichtung verwendet, um in Kombination mit der aus den Sensorsignalen ermittelten Position des Instruments bestimmte Steuersignale an die passiven Aktuatoren abzugeben, um so eine bestimmte Bewegung des schwenkbaren Armes an den jeweiligen Gelenken zu erschweren bzw. zu verlangsamen oder zu erleichtern bzw. zu vereinfachen.

Der schwenkbare Arm gemäß der vorliegenden Erfindung ermöglicht es somit einer Bedienperson ein von dem schwenkbaren Arm geführtes Instrument unter Mithilfe der Steuervorrichtung relativ einfach und genau zu einer gewünschten Position oder entlang eines vorgegebenen Weges zu bewegen oder auch innerhalb eines gewünschten Bereiches zu bewegen, um zum Beispiel ein Verfahren durchzuführen, welches vor dem Einsatz des mit dem schwenkbaren Arm verbundenen Instrumentes geplant wurde.

Da erfindungsgemäß nur passive Aktuatoren, zum Beispiel steuerbare Bremselemente bei den einzelnen Gelenken verwendet werden, wird kein aktives Element wie zum Beispiel ein Motor benötigt, so dass der schwenkbare Arm kostengünstig gefertigt werden kann, eine einfache Mechanik aufweist, einfach angesteuert werden kann und relativ leicht ist. Es wird demzufolge kein Antriebselement für den schwenkbaren Arm benötigt, da die zur Bewegung des schwenkbaren Armes erforderliche Kraft von einer Bedienperson aufgebracht wird und diese Bewegung durch den erfindungsgemäßen schwenkbaren Arm unter Mitwirkung der Steuervorrichtung durch den gezielten und dosierten Einsatz der passiven Aktuatoren zu der gewünschten Position gelenkt wird.

Einer Bedienperson kann somit zum Beispiel eine haptische Rückkopplung oder ein taktiles Feedback gegeben werden, ob beispielsweise die Bewegungsrichtung, in welcher der schwenkbare Arm von der Bedienperson bewegt wird, richtig ist oder nicht. Hierzu kann zum Beispiel eine an das Gelenk durch den Aktuator angelegte Bremskraft erhöht werden, wenn die Bedienperson den schwenkbaren Arm in eine Richtung bewegt, welche nicht einer gewünschten oder geplanten Richtung entspricht und die Bremskraft kann verringert werden wenn die Bewegung des schwenkbaren Armes durch die Bedienperson in die gewünschte Richtung geht. Der erfindungsgemäß an einem oder jedem Gelenk vorgesehene passive Aktuator kann sogar das jeweilige Gelenk vollständig blockieren, wenn bei einer Weiterbewegung des schwenkbaren Armes das mit dem schwenkbaren Arm verbundene Instrument in einen sensiblen Bereich eindringen würde, um so zum Beispiel nicht erwünschte Verletzungen oder Eingriffe zu vermeiden.

Vorteilhaft kann das Feedback so ausgestaltet sein, dass die einer von einem Benutzer initiierten Bewegung vom schwenkbaren Arm entgegengesetzte Kraft so dosiert wird, dass der Benutzer bestimmte Bereich in welchen sich zum Beispiel ein mit dem Arm verbundenes Instrument befindet "fühlen" kann. So können zum Beispiel basierend auf einer vorher durchgeführten Aufnahme eines zu behandelnden Körpers bestimmten Bereichen oder Strukturen, wie zum Beispiel Adern, Sehnen, Knochen, weiches Gewebe usw. durch einen vor der Behandlung durchgeführten Verarbeitungsschritt, zum Beispiel eine geeignete Segmentierung des Körpers, bestimmte Werte einer Widerstandskraft zugeordnet werden, welche einer Bewegung zum Beispiel eines mit dem Arm verbundenen Instruments entgegengesetzt wird, wenn sich beispielsweise die Instrumentenspitze einem solchen Bereich annähert oder in einen Bereich eintritt. Dabei können verschiedenen Strukturen und/oder der näheren Umgebung davon unterschiedliche Widerstandswerte zugeordnet werden, so dass die passiven Aktuatoren des Armes Bewegungen in die Richtung der oder durch die entsprechenden Bereiche hindurch genau dosierte Brems- oder Haltekräfte entgegensetzen. Ein Benutzer kann durch ein solches taktiles Feedback "fühlen" in welchem Bereich sich ein mit dem Arm verbundenes Instrument befindet, indem verschiedenen Bereichen oder Strukturen verschiedene Werte eines Bewegungswiderstandes zugeordnet werden.

Bevorzugt ist der schwenkbare Arm und insbesondere jeder Aktuator so ausgelegt, dass die von einem Menschen aufbringbare Kraft von zum Beispiel 2kN gehalten werden kann, d.h. eine Bedienperson kann ein von dem Aktuator festgestelltes Gelenk auch über eine an Teilelementen des schwenkbaren Arms angreifende Hebelkraft nicht mehr bewegen.

Das mit dem schwenkbaren Arm verbundene Instrument ist bevorzugt ein medizinisches und/oder chirurgisches Instrument und ist direkt oder über einen Adapter oder geeigneten Halter an einem Teilelement des schwenkbaren Armes angebracht, welcher bewegt werden kann, wenn der Arm an einer Haltevorrichtung befestigt ist. So kann beispielsweise ein Nagel, eine Schraube, ein Endoskop, ein Mikroskop, eine Biopsie-Nadel, eine Schneidvorrichtung wie zum Beispiel ein Skalpell, ein Katheter oder eine Katheterführung, ein Bohrer, eine Bohrschablone, ein Schneidblock oder eine Schneidschablone oder ähnliches als Instrument verwendet werden, welches mit dem schwenkbaren Arm verbunden werden kann und durch die Steuervorrichtung in eine gewünschte Position gebracht, entlang einer vorgegebenen Bahn oder in einem bestimmten Bereich bewegt werden kann.

Die Erfindung bezieht sich weiter auf ein System mit einem wie oben beschriebenen schwenkbaren Arm und einer Eingabevorrichtung, um zum Beispiel Steuerdaten für die Steuervorrichtung, wie zum Beispiel die Art und/oder Abmessungen des verwendeten Instruments, die Art des gewünschten durchzuführenden Verfahrens und/oder Daten über das Zielobjekt, zum Beispiel den zu behandelnden oder zu untersuchenden Körper bzw. Körperteil einzugeben, welche beispielsweise über Computertomografie (CT), Kernspinresonanzaufnahmen (MRI), Ultraschalluntersuchungen, Positronen-Emissions-Tomographie (PET), oder andere geeignete Verfahren gewonnen wurden. Somit kann eine Bedienperson anhand von zum Beispiel dreidimensionalen Daten eines zu behandelnden Körperteils, beispielsweise eines Gehirns, vor einem neurochirurgischen Eingriff unter Verwendung einer geeigneten Planungs-Software einen Bereich definieren, in welchem sich ein mit dem schwenkbaren Arm verbundenes Schneidgerät bzw. dessen schneidende Spitze bewegen kann, um so sicherzustellen, dass kein Eingriff in ein außerhalb dieses Bereiches liegendes Gewebe erfolgen kann und nur das Gewebe im Bereich des Tumors und einem vorgegebenen Bereich um den Tumor herum mit dem Schneidgerät entfernt werden kann. Hierzu muss der zu behandelnde Körper bzw. Körperteil registriert werden, d.h. die relative Position zwischen dem schwenkbaren Arm bzw. dem damit verbundenen Instrument und dem zu behandelnden Körper muss erfasst werden. Dies kann zum Beispiel mit passiven Markern erfolgen. Solche Verfahren sind im Stand der Technik bekannt und werden hier nicht näher beschrieben.

Es kann auch das zuvor beschriebene taktile Feedback durch entsprechende Steuerdaten für die Steuervorrichtung realisiert werden, d. h. dass zum Beispiel bestimmten Körperstrukturen bestimmte von den Aktuatoren zu realisierende Widerstand- oder Bremskräfte zugeordnet werden.

Bevorzugt kann bei dem erfindungsgemäßen System eine Anzeige- oder Datenausgabevorrichtung beispielsweise in Form eines Monitors vorgesehen werden, an welchem zum Beispiel positionsbezogene Daten des mit dem schwenkbaren Arm verbundenen Instruments und/oder des zu behandelnden Körpers bzw. Körperteils angezeigt werden, um somit einer Bedienperson anzuzeigen, in welchem Bereich des Körpers sich das Instrument momentan befindet oder wie die nähere oder weitere Umgebung des Behandlungsbereiches aussieht.

Gemäß einem weiteren Aspekt der Erfindung wird ein Verfahren zur Ansteuerung des oben beschriebenen schwenkbaren Arms mit mindestens einem Gelenk mit ansteuerbaren passiven Aktuator vorgeschlagen, so dass ein mit dem schwenkbaren Arm verbundenes Instrument aufgrund der auf mindestens einen passiven Aktuator einwirkenden Steuersignale unter Einwirkung einer äußeren Kraft von einer Bedienperson nur eine oder mehrere vorgegebene Bewegungen ausführen kann, zum Beispiel an eine bestimmte Position geführt werden kann, indem von dieser Position wegführende Bewegungen gebremst und zu dieser Position hinführende Bewegungen von dem mindestens einen passiven Aktuator freigegeben werden, wobei auch ein bestimmter Weg zu dieser Position hin vorgegeben werden kann, welcher von dem Instrument durchfahren werden soll. Weiterhin ist es möglich zum Beispiel einen Aktionsradius für das medizinische Instrument zu definieren, in welchem es bewegt werden kann, wobei der mindestens eine steuerbare passive Aktuator eine Bewegung des Instruments in Richtung auf einen für das Instrument nicht zugänglichen Bereich zum Beispiel zunehmend erschweren oder bremsen kann und zum Beispiel an der Bereichsgrenze eine vollständige Sperrung oder Blockade verursacht.

Das erfindungsgemäße Ansteuerverfahren kann vorteilhaft so ausgestaltet sein, dass eine von einer gewünschten Bewegung abweichende Bewegung um so stärker gebremst wird, je weiter die tatsächliche Bewegung von der gewünschten Bewegung abweicht, wodurch einer Bedienperson eine haptische Rückkopplung gegeben wird.

Vorteilhaft können die Steuereigenschaften bzw. Steueralgorithmen des erfindungsgemäßen Ansteuerverfahrens vor dem Einsatz des schwenkbaren Armes festgelegt werden, zum Beispiel mit einer geeigneten Planungssoftware. Dabei kann zum Beispiel unter Verwendung von dreidimensionalen Daten des Zielobjekts ein Ort oder Bereich in einem zu behandelnden oder untersuchenden Körper bzw. Körperteil vorgegeben werden, an welchem das mit dem schwenkbaren Arm verbundene Instrument positioniert oder bewegt werden soll, wobei auch der Weg vorgegeben werden kann, welchen das Instrument zu der gewünschten Position durchlaufen soll. Dies kann zum Beispiel bei HNO Eingriffen der Fall sein, wenn ein Instrument beispielsweise durch ein Nasenloch zu einer vorgegebenen Position geführt werden soll. Es können für Eingriffe im Bereich der Hüfte oder des Knies, sowie bei der spinalen Chirurgie auch bestimmte Elemente, wie zum Beispiel Schrauben, Nägel, Bohrer, Schneidwerkzeuge, Bohrschablonen oder Schneidblöcke an gewünschte Stellen des zu behandelnden Körperteils geführt werden, um beispielsweise eine Schraube an einer gewünschten Position anzulegen, so dass sie von einer Bedienperson leicht in der richtigen Position eingeschraubt werden kann. Weiterhin ist es auch möglich sogenannte verbotene Zonen ("no-go-zones") zu definieren, in welche das mit dem schwenkbaren Arm verbundene Instrument nicht eingreifen darf, zum Beispiel bei chirurgischen Eingriffen, welche nur in einem bestimmten Bereich erfolgen sollen und bei welchen sichergestellt sein soll, dass angrenzende benachbarte Bereiche nicht von dem Eingriff betroffen sind, wie zum Beispiel bei der Neurochirurgie.

Eine Ausführungsform des erfindungsgemäßen Verfahrens wird prinzipiell anhand einer beispielhaften Ausführungsform veranschaulicht. Es zeigt:

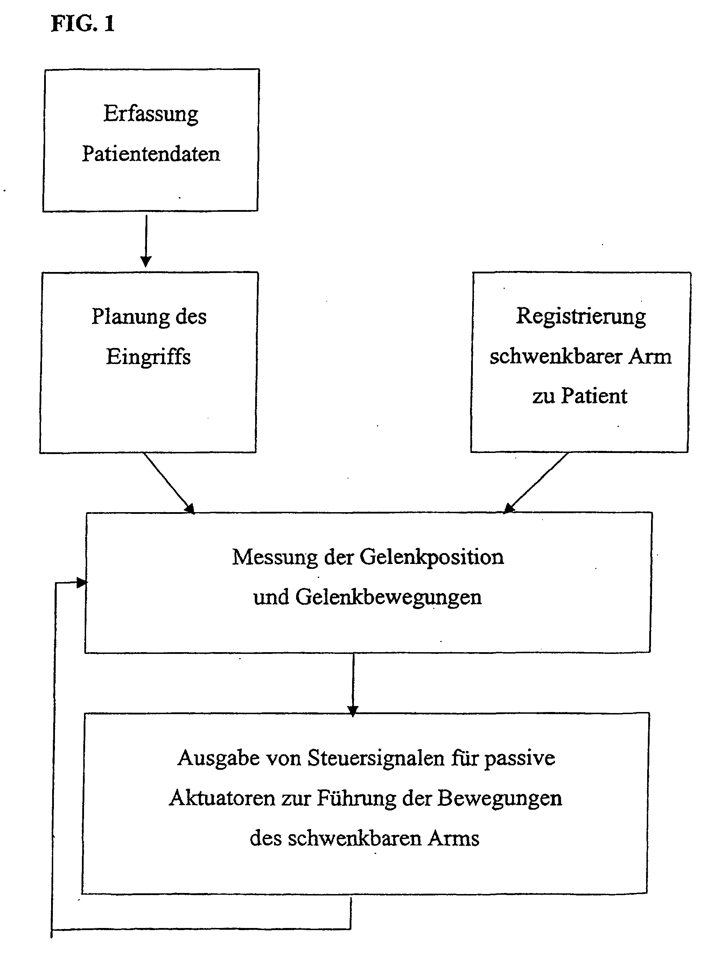

- Figur 1: ein Ablaufdiagramm zur Vorbereitung und Durchführung der Ansteuerung eines schwenkbaren Arms.

Wie in Figur 1 gezeigt, werden in einem ersten Schritt Patientendaten mittels geeigneter Verfahren, wie zum Beispiel CT, MR, PET oder anderen Verfahren erfasst, um hierdurch zum Beispiel dreidimensionale Daten eines zu untersuchenden oder zu behandelnden Objektes oder Körperteils zu erhalten. Basierend auf diesen erfassten Patientendaten wird bevorzugt unter Verwendung einer geeigneten Planungs-Software ein tatsächlich vorzunehmender Eingriff geplant, wobei beispielsweise bestimmte am zu behandelnden Körper zu durchfahrende Bahnen oder anzufahrende Positionen oder auch Bereiche vorgegeben werden können, in welche nicht eingegriffen werden darf.

Der schwenkbare Arm wird zu dem Patienten registriert, d.h. der Patient und der schwenkbare Arm werden in ein zueinander definiertes Lageverhältnis gebracht oder es wird zum Beispiel anhand von mit einem Patienten verbundenen Markern die genaue Position des Patienten ermittelt, wobei der schwenkbare Arm an einem dazu feststehenden Punkt befestigt ist oder wird.

Basierend auf den durch die Planung des Eingriffs vorgegebenen gewünschten oder unerwünschten Bewegungen bzw. Bewegungsrichtungen des schwenkbaren Arms und unter Verwendung der Patientendaten erfolgt nun kontinuierlich eine Messung der Positionen oder Bewegungen der einzelnen Gelenke des schwenkbaren Armes, wobei in Abhängigkeit von dem gewünschten durchzuführenden Eingriff Steuersignale an die bei den Gelenken vorgesehenen passiven Aktuatoren ausgegeben werden, um die Bewegungen des schwenkbaren Armes durch eine Bedienperson zu führen, d.h. es können zum Beispiel Bewegungen einzelner Gelenke in unerwünschte Richtungen gebremst oder blockiert werden und Bewegungen in eine oder mehrere gewünschte oder erlaubte Richtungen können von den passiven Aktuator freigegeben werden.

## Patentansprüche

1. Schwenkbarer Arm mit:
a) einer Befestigungsvorrichtung für ein Instrument um ein Zielobjekt zu untersuchen oder zu behandeln;
b) mindestens einem Gelenk um die Befestigungsvorrichtung für das Instrument zu bewegen;
c) mindestens einem Sensor an dem mindestens einen Gelenk, um die Position und/oder Bewegung des mindestens einen Gelenkes zu erfassen;
d) einem steuerbaren passiven Aktuator für das mindestens eine Gelenk, um eine Bewegung des Gelenkes bremsen und/oder verhindern zu können; und
e) einer Steuervorrichtung, welche die relative Position zwischen dem schwenkbaren Arm und dem Zielobjekt erfasst und den steuerbaren passiven Aktuator basierend auf den von dem mindestens einen Sensor übermittelten Positions- und/oder Bewegungsdaten und basierend auf Daten des Zielobjekts ansteuert.

2. Schwenkbarer Arm nach Anspruch 1, wobei das Instrument ein medizinisches und/oder chirurgisches Instrument, insbesondere ein Nagel, eine Schraube, ein Endoskop, ein Mikroskop, eine Schneidvorrichtung, ein Skalpell, eine Biopsie-Nadel, ein Katheter, eine Katheterführung, ein Bohrer, eine Bohrschablone oder eine Schneidschablone ist.

3. Schwenkbarer Arm nach einem der vorhergehenden Ansprüche, wobei das mindestens eine Gelenk eine Drehbewegung und/oder eine Klappbewegung ermöglicht.

4. Schwenkbarer Arm nach einem der vorhergehenden Ansprüche, wobei zwei, drei, vier, fünf oder mehr Gelenke am schwenkbaren Arm vorgesehen sind.

5. Schwenkbarer Arm nach einem der vorhergehenden Ansprüche, wobei der mindestens eine steuerbare passive Aktuator an dem Gelenk so ausgelegt ist, dass er die von einem Menschen auf das Gelenk angelegte Kraft halten kann.

6. System mit einem schwenkbaren Arm nach einem der vorhergehenden Ansprüche und einer Eingabevorrichtung zur Eingabe von Daten eines von dem Instrument zu untersuchenden und/oder zu behandelnden Zielobjekts und/oder zur Eingabe von Steuerdaten für ein durchzuführendes Verfahren.

7. System nach Anspruch 6 mit einer Anzeigevorrichtung, insbesondere einem Monitor zur Anzeige von Positionsdaten und/oder einer Umgebung des durch den schwenkbaren Arm geführten Instruments.

8. Verfahren zur Ansteuerung eines schwenkbaren Arms oder Systems nach einem der vorhergehenden Ansprüche, wobei der mindestens eine steuerbare passive Aktuator so angesteuert wird, dass ein mit dem schwenkbaren Arm verbundenes Instrument nur eine oder mehrere vorgegebene Bewegungen ausführen kann.

9. Verfahren nach Anspruch 8, wobei vor der Ansteuerung der passiven Aktuatoren des schwenkbaren Armes die durch das Steuerverfahren freizugebenden Bewegungen oder Bewegungsbereiche vorgegeben werden.

10. Verfahren nach Anspruch 8 oder 9, wobei dreidimensionale Daten des von dem Instrument zu untersuchenden oder zu behandelnden Zielobjektes für die Ansteuerung und/oder Planung der Ansteuerung, insbesondere zur Realisierung eines taktilen Feedbacks, verwendet werden.

11. Verfahren nach einem der Ansprüche 8 bis 10, wobei die Ansteuerung so erfolgt, dass einer Bedienperson eine haptische Rückkopplung gegeben wird.

## Claims

1. A pivotable arm, comprising:
a) a fixing device for an instrument, to examine or treat a target object;
b) at least one pivot, to move said fixing device for said instrument;
c) at least one sensor on said at least one pivot, to detect the position and/or movement of the at least one pivot;
d) a controllable, passive actuator for the at least one pivot, in order to be able to brake and/or prevent a movement of the pivot; and
e) a control device which detects the relative position of the pivotable arm and the target object and guides the controllable, passive actuator on the basis of the position data and/or movement data transmitted from the at least one sensor and based on data of the target object.

2. The pivotable arm according to claim 1, wherein the instrument is a medical and/or surgical instrument, in particular a nail, a screw, an endoscope, a microscope, a cutting device, a scalpel, a biopsy needle, a catheter, a catheter guide, a drill, a drilling template or a cutting template.

3. The pivotable arm according to any one of the preceding claims, wherein the at least one pivot enables a rotational movement and/or a tilting movement.

4. The pivotable arm according to any one of the preceding claims, wherein two, three, four, five or more pivots are provided on the pivotable arm.

5. The pivotable arm according to any one of the preceding claims, wherein the at least one controllable, passive actuator on the pivot is designed in such a way that it can hold the force applied to the pivot by a person.

6. A system comprising a pivotable arm according to any one of the preceding claims and an input device for inputting data of a target object to be examined and/or treated by the instrument and/or for inputting control data for a method to be performed.

7. The system according to claim 6, comprising a display means, in particular a monitor, for displaying position data and/or a vicinity of the instrument guided by the pivotable arm.

8. A method for guiding a pivotable arm or a system according to any one of the preceding claims, wherein the at least one controllable, passive actuator is guided in such a way that an instrument connected to the pivotable arm can perform only one or a number of predetermined movements.

9. The method according to claim 8, wherein the movements or movement areas to be released by the control method are predetermined prior to guiding the passive actuators of the pivotable arm.

10. The method according to claim 8 or claim 9, wherein three-dimensional data of the target object to be examined or treated by the instrument are used for guiding and/or for guidance planning, in particular for realising a tactile feedback.

11. The method according to any one of claims 8 to 10, wherein the pivotable arm is guided in such a way that an operator is given a haptic feedback.

## Revendications

1. Bras pivotant avec:
a) un dispositif de fixation pour un instrument servant à examiner ou traiter un objet cible;
b) au moins une articulation pour déplacer le dispositif de fixation de l'instrument;
c) au moins un capteur sur l'au moins une articulation, pour saisir la position et/ou le mouvement de l'au moins une articulation;
d) un actionneur passif manoeuvrable pour l'au moins une articulation, afin de pouvoir freiner et/ou empêcher un mouvement de l'articulation; et
e) un dispositif de commande qui saisit la position relative du bras pivotant et de l'objet cible et commande l'actionneur passif manoeuvrable sur base des données de position et/ou de mouvement transmises par l'au moins un capteur et sur base de données de l'objet cible.

2. Bras pivotant suivant la revendication 1, l'instrument étant un instrument médical et/ou chirurgical, en particulier un clou, une vis, un endoscope, un microscope, un dispositif de coupe, un scalpel, une aiguille à biopsie, un cathéter, un guide de cathéter, un foret, un gabarit de perçage ou un gabarit de coupe.

3. Bras pivotant suivant l'une des revendications précédentes, l'au moins une articulation permettant un mouvement de rotation et/ou un mouvement de basculement.

4. Bras pivotant suivant l'une des revendications précédentes, deux, trois, quatre, cinq articulations ou plus étant prévues sur le bras pivotant.

5. Bras pivotant suivant l'une des revendications précédentes, l'au moins un actionneur passif manoeuvrable sur l'articulation étant conçu de telle façon qu'il peut contenir la force exercée par une personne sur l'articulation.

6. Système avec un bras pivotant suivant l'une des revendications précédentes et un dispositif d'introduction pour introduire des données d'un objet cible à examiner et/ou traiter avec l'instrument et/ou introduire des données de commande pour un procédé à réaliser.

7. Système suivant la revendication 6 avec un dispositif d'affichage, en particulier un moniteur pour l'affichage de données de position et/ou d'un environnement de l'instrument guidé par le bras pivotant.

8. Procédé de commande d'un bras pivotant ou d'un système suivant l'une des revendications précédentes, l'au moins un actionneur passif manoeuvrable pouvant être commandé de telle façon qu'un instrument relié au bras pivotant peut exécuter uniquement un ou plusieurs mouvements prédéfinis.

9. Procédé suivant la revendication 8, les mouvements ou plages de mouvement à libérer par le procédé de commande étant prédéfinis avant la commande des actionneurs passifs du bras pivotant.

10. Procédé suivant la revendication 8 ou 9, des données tridimensionnelles de l'objet cible à examiner ou traiter avec l'instrument étant utilisées pour la commande et/ou la planification de la commande, en particulier pour la réalisation d'une rétroaction tactile.

11. Procédé suivant l'une des revendicatio ns 8 à 10, la commande se faisant de telle façon qu'une rétroaction haptique est donnée à un opérateur.
